# EUROPEAN PATENT APPLICATION

(11) **EP 0 530 410 A1**
(43) Date of publication of application: **10.03.1993**
(21) Application number: 91308027.1
(22) Date of filing: 02.09.1991
(51) Int. Cl.: B21G 1/08, A61B 17/06, B23K 26/00, B23H 9/08

(54) **Method of producing surgical needle with gut**

(71) Applicant: MATSUTANI SEISAKUSHO CO. LTD.,, Takanezawa-Machi Shioya-Gun Tochigi-ken (JP)
(72) Inventor: Matsutani, Kanji, Shioya-gun, Tochigi-Ken (JP); Otsuka, Tadashi, Shioya-gun, Tochigi-Ken (JP); Saito, Masahiko, Utsunomiya-shi, Tochigi-ken (JP)
(74) Representative: Votier, Sidney David

(57) **Abstract**

There is provided an optimum method of producing, particularly, a thin, short surgical needle (3) with a gut (2). A pointed end (1b) of the surgical needle (3) is formed at a final step of the production method in order to prevent damage to the pointed end (1b) during the production of the surgical needle (3). An elongated base element (1) longer than the surgical needle (3) is prepared, and after the formation of a hole (1a) in the base element (1), the attachment of the gut (2) to the base element (1), and the bending of the base element (1) are carried out, the base element (1) is cut into a length (Lx) substantially equal to the length of the surgical needle (3), and the cut end of the base element (1) is formed into a pointed configuration (1b).

## Description

### BACKGROUND OF THE INVENTION

This invention relates to a method of producing a surgical needle with a gut.

One conventional method of producing a surgical needle with a gut is disclosed as the prior art in U. S. Patent No. 5,012,066 (corresponding to Japanese Laid-Open (Kokai) Patent Application No. 90237/91), and such a method is disclosed also in Japanese Laid-Open Patent Application No. 212027/88 and U. S. Patent No. 4,910,377 (corresponding to Japanese Laid-Open Patent Application Nos. 130888/89 and 52189/90). More specifically, such disclosed method comprises the steps of forming one end of an elongated base element into a pointed shape, subsequently forming a hole in the other end face of the base element, subsequently inserting one end portion of a gut into this hole and press-deforming the other end portion of the base element to attach the gut to the base element, and subsequently bending or curving the base element.

In the above conventional method, however, since the pointed end is formed at the first step, the pointed end is quite liable to be damaged during the subsequent gut-attaching step or the subsequent bending step. More specifically, at the gut-attaching step, when the other end portion of the base element is to be press-deformed, that portion of the base element adjacent to the pointed end thereof is grasped by a jig, and therefore the pointed end is liable to be damaged by this jig. At the bending step, the base element is bent up to the pointed end thereof, and therefore the pointed end is liable to be damaged.

Further, during storage and transport of the base elements whose one ends have already been pointed, the pointed ends have often been damaged by contact between the base elements.

Although it is mentioned in the above U. S. Patent No. 4,910,377 that the pointed end may be formed after the formation of the hole, there is no mention of the formation of the pointed end after the gut-attaching step and the bending step.

Japanese Patent Publication No. 35151/85 describes in Fig. 3 a method in which a hole is formed in one end face of an elongated base element longer than the length of a final product (surgical needle), and then the base element is cut in such a manner that the length of the thus-cut base element from the one end face thereof to the cut end is substantially equal to the length of the surgical needle of a final form, and then this cut end is formed into a pointed configuration having cutting edges. Although no mentioned is made of the bending of the base element and the attachment of a gut, it is surmised from the drawings that these steps are carried out after the formation of the pointed end. Therefore, with this method, also, the above disadvantages are not overcome.

Japanese Laid-Open Patent Application No. 17339/84 discloses a method in which one end of a base element, having a diameter of not more than 200 µm, is pointed by electropolishing. In this publication, there is disclosed a method in which first, the opposite ends of the base element are pointed, and then the base element is cut into a required length, and then a hole is formed in an end face formed by the above cutting, and then the base element is bent. No mention is made of the attachment of a gut. It is beyond doubt that the attachment of the gut and the bending of the base element are carried out after the formation of the pointed end. Therefore, with this method, also, the above disadvantages are not overcome.

Referring to another problem, the shorter the surgical needle, the more difficult the holding of the base element when attaching the gut thereto, and it is also difficult to bend the base element. This problem has not yet been overcome by the above prior art. In the methods disclosed in Japanese Patent Publication No. 35151/85 and Japanese Laid-Open Patent Application No. 17339/84, the base element longer than the length of the surgical needle (final product) is used, and the base element is cut to the length of the surgical needle before the attachment of the gut and the bending of the base element are carried out.

### SUMMARY OF THE INVENTION

It is an object of this invention to provide a method of producing a surgical needle with a gut which method prevents a pointed end of a base element from being damaged during the production of the surgical needle.

Another object of the invention is to provide a method by which a thin, short surgical needle with a gut can be easily produced.

According to one aspect of the present invention, there is provided a method of producing a surgical needle with a gut, comprising the steps of:
(a) forming a hole in one end face of an elongated base element along an axis of the base element;
(b) inserting one end portion of the gut into the hole and fixing the one end portion of the gut relative to the hole to thereby attach the gut to the base element; and
(c) pointing the other end of the base element remote from the hole;
(d) the steps (a), (b) and (c) being carried out in this order.

According to another aspect of the invention, there is provided a method of producing a surgical needle with a gut, comprising the steps of:
(a) forming a hole in one end face of an elongated base element along an axis of the base element, the base element being greater in length than the surgical needle;
(b) inserting one end portion of the gut into the hole and fixing the one end portion of the gut relative to the hole to thereby attach the gut to the base element;
(c) cutting the base element in such a manner that a length of the thus cut base element from the one end face to the cut end thereof is substantially equal to the length of the surgical needle; and
(d) pointing the cut end of the base element remote from the hole;
(e) the steps (a), (b), (c) and (d) being carried out in this order.

According to a further aspect of the invention, there is provided a method of producing a surgical needle with a gut, comprising the steps of:
(a) forming a hole in one end face of an elongated base element along an axis of the base element, the base element being greater in length than the surgical needle;
(b) inserting one end portion of the gut into the hole and fixing the one end portion of the gut relative to the hole to thereby attach the gut to the base element;
(c) bending at least part of that portion of the base element extending from the one end face over a length substantially equal to the length of the surgical needle;
(d) cutting the base element in such a manner that a length of the thus cut base element from the one end face to the cut end thereof is substantially equal to the length of the surgical needle; and
(e) pointing the cut end of the base element remote from the hole;
(f) the step (a) being carried out before the steps (b) and (c), and the steps (d) and (e) being carried out in this order after the steps (b) and (c).

### BRIEF DESCRIPTION OF THE DRAWINGS

Fig. 1 is a flow chart of a process for the production of a surgical needle with a gut according to the present invention;
Fig. 2 is a view of a number of base elements joined together by a tape;
Fig. 3 is a view showing the manner of forming a hole in each base element;
Fig. 4 is a view of the base element to which a gut has been attached;
Fig. 5 is a view showing the manner of bending the base element;
Fig. 6 is a view of the base element subjected to a bending operation;
Fig. 7 is a view of the base element subjected to a cutting operation;
Fig. 8 is a view of the base element subjected to a pointing operation, that is, a surgical needle (final product); and
Fig. 9 is a view showing the surgical needle mounted on a holder plate.

### DESCRIPTION OF THE PREFERRED EMBODIMENT

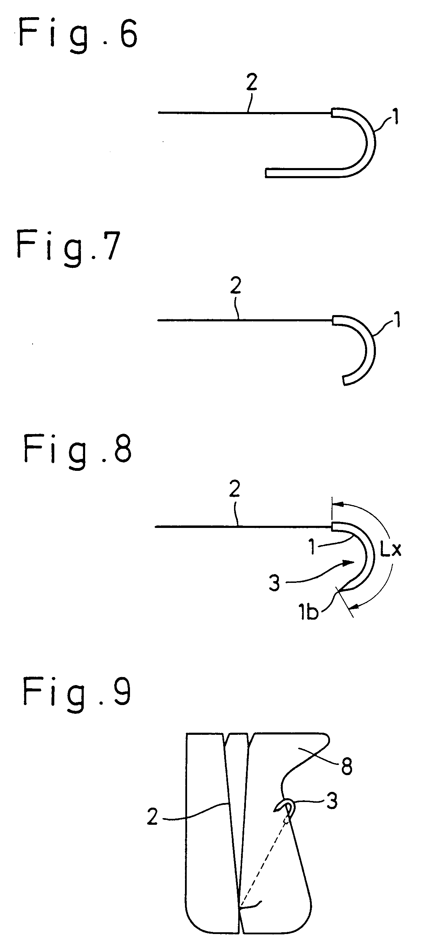

A method of producing a taper point-type surgical needle with a gut, provided in accordance with the present invention, will now be described with reference to the drawings. The surgical needle has a diameter of not more than 100 µm and a length Lx (see Fig. 8) of not more than 6.5 mm. For example, where the diameter is 100 µm, the length Lx is in the range of 3.5 to 6.5 mm. Where the diameter is 50 µm, the length Lx is in the range of 2.5 to 4.5 mm. Where the diameter is 30 µm, the length Lx is in the range of 1.5 to 3.5 mm.

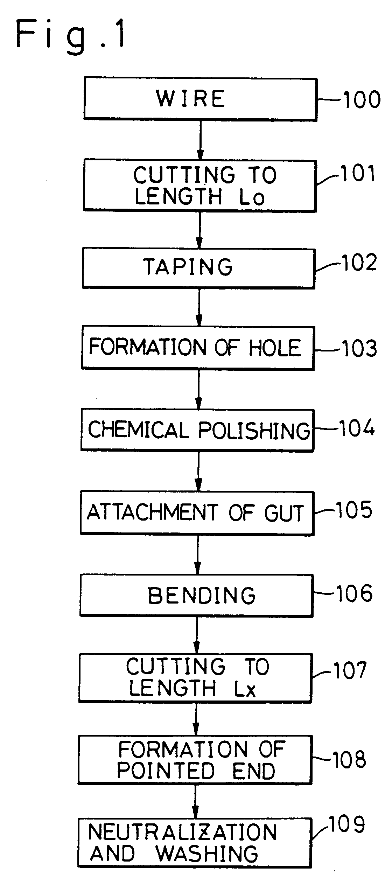

The entire process of this production method is shown in Fig. 1. First, a wire of stainless steel is prepared (Step 100). This wire is cut into a predetermined length Lo to provide a number of thin straight base elements 1 (Step 101). The diameter of the base element 1, which is shown in an exaggerated manner in the drawings, is substantially equal to or slightly larger than the diameter of the surgical needle. The length Lo of the base element 1 is, for example, 20 to 30 mm, and is considerably larger than the length Lx of the surgical needle.

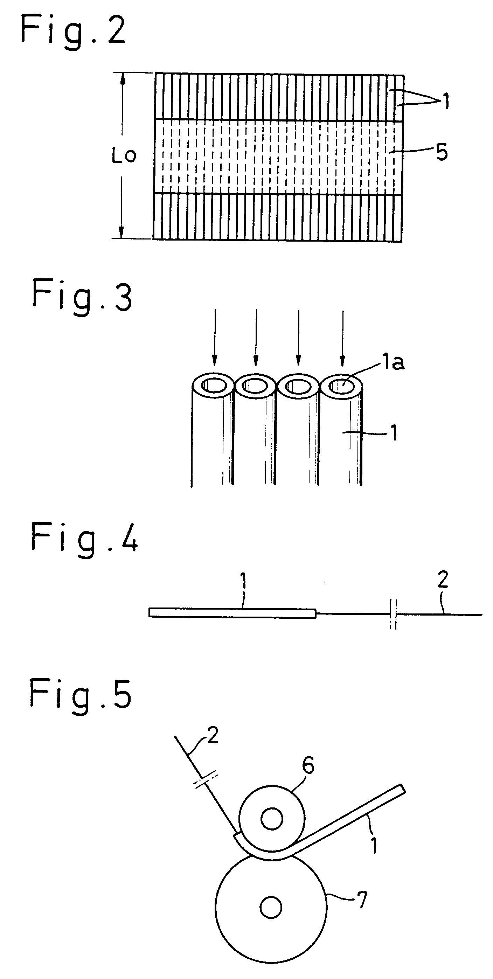

Next, as shown in Fig. 2, a number of base elements 1 are juxtaposed in a common plane, and are joined together into a bundle by an adhesive tape 5 (Step 102). Then, the bundle of base elements 1 is held by a holder (not shown), and in this condition a laser beam or an electron beam is applied sequentially to one end faces of the base elements 1 to form a hole 1a in the one end face of each base element 1, as shown in Fig. 3 (Step 103).

Then, the adhesive tape 5 is removed from the base elements 1, and the base elements 1 are subjected to chemical polishing (Step 104). This chemical polishing is carried out mainly in order to remove an oxide film formed on the surface of the base element 1, to remove burrs formed on the edge of the hole 1a defining an inlet of the hole 1a, and to effect a fine adjustment of the diameter of the base element 1.

Then, as shown in Fig. 4, one end portion of a gut 2 is inserted into the hole 1a formed in one end portion of the base element 1, and then the one end portion of the base element 1 is press-deformed by a pair of mold members having respective grooves of a semi-circular shape to thereby fixedly secure the gut 2 to the base element 1 (Step 105).

Then, the base element 1 is bent or curved by a conventional method shown in Fig. 5 (Step 106). More specifically, a rigid roller 6 and an elastic roller 7 movable toward and away from the rigid roller 6 are prepared. The base element 1 is disposed between the two rollers 6 and 7, and in this condition the roller 7 is urged toward the roller 6. Then, the roller 7 is rotated to cause the roller 6 to rotate. As a result, the base element 1 is bent or curved. As shown in Fig. 6, the bent portion of the base element 1, which starts from the one end face thereof in which the hole 1a is formed, is substantially equal to the length Lx of the surgical needle (final product). The bent portion starting from the one end face may be shorter or longer than the length Lx of the surgical needle. Also, the bent portion may exclude the one end portion of the base element 1 in which the hole 1a is formed.

As described above, since the length Lo of the base element 1 is sufficiently long, the holding of the base element 1 when attaching the gut 2 thereto, as well as the bending of the base element 1, can be effected easily.

Then, as shown in Fig. 7, the base element 1 is cut in such a manner that the length of the thus-cut base element 1 from the one end face to this cut end is longer several µm or several tens of µm than the length Lx of the surgical needle (Step 107). Then, using an electropolishing process as disclosed in Japanese Laid-Open Patent Application No. 17339/84, the other end (cut end) of base element 1a remote from the hole 1a is pointed as at 1b, as shown in Fig. 8 (Step 108). More specifically, an electrolyte, composed of an aqueous solution of phosphoric acid and dichromic acid, is used. A positive terminal of a power source is connected to the base element 1 whereas a negative terminal thereof is connected to an electrode rod. The electrode rod is dipped in the electrolyte, and in this condition the cut end of the base element 1 is contacted with the electrolyte for a short period of time. By doing so, the cut end of the base element 1 is formed into a pointed configuration as at 1b. Then, that portion of the base element 1 including the pointed end 1b is neutralized, and is washed (Step 109).

By doing the above steps, the production of the surgical needle 3 with the gut 2 is finished. Thereafter, the surgical needle 3 is hooked on an edge of a holder plate 8 of a unique shape as shown in Fig. 9, and the gut 2 is wound on the holder plate 8, and then the holder plate 8 with the surgical needle 3 is packaged.

In the above method, the formation of the pointed end is carried out at the final step, and therefore damage to the pointed end will not occur at the gut-attaching step and the bending step. The base element is bent after the gut is attached to the base element longer than the surgical needle (final product), and therefore these operations can be carried out easily and accurately even if the length of the surgical needle is short.

The present invention is not limited to the above embodiment, and various modifications can be made. For example, the gut may be attached after the bending step. Also, the bending step may be omitted. In the above embodiment, although the pointed end is formed by electropolishing, the formation of the pointed end may be effected by chemical polishing using a strong acid, sand blasting, or any other suitable method. In the above embodiment, although the gut is fixedly attached to the base element by press-deforming, this may be done by the use of an adhesive.

## Claims

1. A method of producing a surgical needle with a gut, comprising the steps of:
(a) forming a hole (1a) in one end face of an elongated base element (1) along an axis of said base element;
(b) inserting one end portion of the gut (2) into said hole and fixing said one end portion of said gut relative to said hole to thereby attach said gut to said base element; and
(c) pointing the other end (1b) of said base element remote from said hole;
CHARACTERIZED in that said steps (a), (b) and (c) are carried out in this order.

2. A method of producing a surgical needle with a gut, comprising the steps of:
(a) forming a hole (1a) in one end face of an elongated base element (1) along an axis of said base element, said base element being greater in length than the surgical needle (3);
(b) inserting one end portion of the gut (2) into said hole and fixing said one end portion of said gut relative to said hole to thereby attach said gut to said base element;
(c) cutting said base element in such a manner that a length of the thus cut base element from said one end face to the cut end thereof is substantially equal to the length of the surgical needle; and
(d) pointing the cut end (1b) of said base element remote from said hole;
CHARACTERIZED in that said steps (a), (b), (c) and (d) are carried out in this order.

3. A method of producing a surgical needle with a gut, comprising the steps of:
(a) forming a hole (1a) in one end face of an elongated base element (1) along an axis of said base element, said base element being greater in length than the surgical needle (3);
(b) inserting one end portion of the gut (2) into said hole and fixing said one end portion of said gut relative to said hole to thereby attach said gut to said base element;
(c) bending at least part of that portion of said base element extending from said one end face over a length substantially equal to the length of the surgical needle;
(d) cutting said base element in such a manner that a length of the thus cut base element from said one end face to the cut end thereof is substantially equal to the length of the surgical needle; and
(e) pointing the cut end (1b) of said base element remote from said hole;
CHARACTERIZED in that said step (a) are carried out before said steps (b) and (c), and said steps (d) and (e) are carried out in this order after said steps (b) and (c).

4. A method according to claim 1, claim 2 or claim 3, in which said pointed end (1b) of said base element (1) is formed by electropolishing.

5. A method according to claim 1, claim 2 or claim 3, in which said surgical needle (3) has a diameter of not more than 100 µm.
